# EUROPEAN PATENT APPLICATION

(11) **EP 4 445 927 A1**
(43) Date of publication of application: **16.10.2024**
(21) Application number: 22903891.4
(22) Date of filing: 24.10.2022
(51) Int. Cl.: A61M 5/14

(54) **INJECTION TOOL FOR ENDOSCOPE**

(30) Priority: 10.12.2021 JP 2021200673
(71) Applicant: Lake R&D Inc., Okaya-shi, Nagano 394-0028 (JP)
(72) Inventor: OGUCHI, Yuji, Okaya-shi Nagano 394-0028 (JP)
(74) Representative: Schweiger, Martin
(86) International application number: PCT/JP2022/039593
(87) International publication number: WO 2023/105958

(57) **Abstract**

An injection tool for an endoscope includes an operation unit main body having an outer tube attached to a distal end thereof, and a slider fixed to a base end of an inner tube having a needle body fixed to a distal end thereof and inserted into the operation unit main body to slide. A guide groove for second protrusion guiding a second protrusion disposed in the distal end of the slider is opened to the operation unit main body, and a locked protrusion of the slider comes into contact with a locking protrusion disposed in the guide groove for second protrusion, thereby preventing unnecessary protrusion of the needle body. When the slider is further pressed into the operation unit main body, the locked protrusion gets over the locking protrusion to cancel the movement prevention of the slider. The needle body is maintained to be housed in the outer tube.

## Description

### TECHNICAL FIELD

The present invention relates to an injection tool for an endoscope which is inserted into a body cavity through a channel open to an insertion unit of the endoscope, is punctured into a submucosal layer of a living body, and injects a treatment liquid.

### BACKGROUND ART

Generally, an endoscopic mucosa resection treatment (EMR) and an endoscopic submucosal layer desquamation treatment (ESD) of resecting a mucosa such as an esophagus, a stomach and a large bowel set a submucosal layer in a floating state by injecting a drug solution (hereinafter, refer to as a treatment liquid) such as a physiological saline solution or a hyaluronic acid with the used of an injection tool for an endoscope in the submucosal layer, and resect the mucosa with a high frequency knife or a high frequency snare.

The injection tool for the endoscope is provided with a first assembly including an outer tube (sheath) which is inserted into a channel open to an insertion unit of the endoscope, and an operation unit main body which is gripped by an operator, and a second assembly including an inner tube which is provided with a needle body in a distal end thereof, and a slider which is relatively moved with respect to the operation unit main body by the operator, and inserting the inner tube provided with the needle body in the distal end thereof into the outer tube from a base end side of the first assembly, and is adapted to allow the needle body to project and set from the distal end of the outer tube by an operation of the operator for moving forward and backward the slider with respect to the operation unit main body. The operation unit main body and the slider construct an operation unit.

In the injection tool for the endoscope, in a state in which the needle body is positioned within the outer tube and the operation unit main body and the slider are relatively fixed in the base end side of the endoscope, the outer tube is inserted into the channel of the insertion unit and the distal end of the outer tube is guided to an affected area on the basis of an image monitoring of a body cavity which is obtained through a lens disposed in the distal end portion of the endoscope.

In this state, the injection tool for the endoscope is used in such a manner as to allow the needle body to protrude out of the outer tube and to be punctured to the affected area by the slider which is pushed to the operation unit main body by the operator, and inject (treat) the treatment liquid injected from a syringe barrel installed to the base end of the slider to the submucosal layer in the affected area from the needle body. When the slider is moved by the operator so that the slider is away from the operation unit main body after this treatment, the injection tool for the endoscope houses the needle body within the outer tube, and is further operated so as to separate the outer tube from the submucosal layer in the affected area by pulling out the operation unit from the channel of the endoscope in a state in which the operation unit main body and the slider are relatively fixed.

### CITATION LIST

### PATENT LITERATURE

Patent Literature 1: Japanese Unexamined Utility Model Publication No. H02-139649
Patent Literature 2: Japanese Unexamined Utility Model Publication No. H01-68052
Patent Literature 3: Japanese Unexamined Patent Publication No. 2020-185037

### SUMMARY OF INVENTION

### TECHNICAL PROBLEM

In the injection tool for the endoscope mentioned above, it is important that the needle body is held in a state in which the needle body protrudes out of the outer tube when injecting the treatment liquid from the puncture, and is held in a state in which the needle body is retracted within the outer tube at the other times.

Describing in detail, in the injection tool for the endoscope, it is necessary that the needle body comes to a state in which the needle body protrudes at a predetermined length out of the outer tube by moving the slider with respect to the operation unit main body to press to the distal end side, and the needle body is housed within the outer tube by moving the slider with respect to the operation unit main body to retract to the base end side.

As described above, it is necessary for the injection tool for the endoscope to be locked in such a manner as to prevent the slider from being pressed against the operation unit main body (to be maintained in such a manner as to prevent the needle body from protruding out of the outer tube) before the treatment before the distal end of the outer tube is positioned in the affected area (the submucosal layer of the esophagus, the stomach or the large bowel). In a case where the state in which the slider is moved with respect to the operation unit main body to be retracted to the base end side is not maintained before the treatment, there is a concern that the slider and the inner tube move to the distal end side by gravity and the needle body protrudes out of the distal end of the outer tube in the injection tool for the endoscope.

Describing more particularly, for example, in a case where the operator holds the injection tool for the endoscope in a state in which the operation unit main body is in a lower side and the slider is in an upper side, or a case where a hook is put on a flange part of the operation unit main body to be suspended in a state in which the operation unit main body is in a lower side and the slider is in an upper side so that a technologist can easily pick during an operation, the slider moves downward by gravity and the needle body may come out of the outer tube. In addition, when the injection tool for the endoscope comes into conflict with something, the slider is pushed and the needle body may come out of a sheath.

As mentioned above, the injection tool for the endoscope is dangerous before being passed through the channel of the endoscope if the needle body comes out of the outer tube. Further, there is a problem that the needle body comes into contact with the inner surface of the channel when being passed through the channel of the endoscope, and the needle body sticks to the other portions than the affected area on its own within the body cavity.

Therefore, for the injection tool for the endoscope, a doctor hopes to hold a state in which the needle body is put out from the distal end of the outer tube only when the injection tool applies treatment to the affected area, and maintain a state in which the needle body is housed within the outer tube at the other times, and cancel the housing maintaining state by intentionally pushing.

In the injection tool for the endoscope described in the patent literature 1, an inner peripheral surface of an operation unit main body is formed into a taper shape toward a distal end (such a shape that a diameter of a structure is inclined toward the distal end), and an outer peripheral surface of a slider is also formed into a taper shape. Thus, both the taper surfaces come into contact and can be fixed by friction when the slider is pressed into the operation unit main body. Therefore, it is possible to hold a protruding state of the needle body. However, the injection tool for the endoscope described in the patent literature 1 is not provided with a mechanism which maintains the state in which the needle body is retracted into the outer tube.

Further, the injection tool for the endoscope described in the patent literature 1 fixes the state in which the needle body is protruded out of the outer tube due to the friction between the inner peripheral surface of the operation unit main body and the outer peripheral surface of the slider. Therefore, the protrusion of the needle body may be insufficient according to a strength of a force applied by an operator (a doctor or a technologist), and there is a problem that a fixing position is hard to be known.

The injection tool for the endoscope described in the patent literature 2 is provided with an operation unit main body which is open in a longitudinal direction of a side wall and includes slide grooves with a plurality of hook-shaped lock grooves in an opening manner, and an inner handle which slides within the operation unit main body and is provided in a side surface thereof with a protrusion fitted to the slide groove and protruding, the needle body disposed in the distal end of the inner handle is slid within the outer tube by sliding the protrusion along the slide groove, and the position of the needle body can be locked by fitting the protrusion to the hook-shaped lock groove.

However, in the injection tool for the endoscope described in the patent literature 2, an operation for rotating the slider is required when moving the protrusion of the slider to the hook-shaped lock groove from the groove in the longitudinal direction. Therefore, there is a problem that the operation can not be smoothly performed. Further, in the injection tool for the endoscope described in the patent literature 2, any specific method for attaching the outer tube to the operation unit main body is not disclosed. Therefore, there is a problem that a safety lacks due to deterioration of an adhesive agent when attaching with the use of the adhesive agent.

The injection tool for the endoscope described in the patent literature 3 is locked in such a manner as to cancel a state in which the needle body protrudes out of the outer tube when the treatment liquid is injected from the puncture, and an operation feeling at this time can be sensed by the doctor, and the outer tube can be securely attached to the operation unit main body.

An object of the present invention is to provide an injection tool for an endoscope which is locked in such a manner as to cancel a state in which a needle body protrudes out of an outer tube when injecting the treatment liquid from the puncture, an operation feeding at this time is sensed by a doctor, it is possible to maintain and cancel a state in which the needle body is housed within the outer tube before the puncture and after injecting the treatment liquid, and an operation feeling when maintaining and cancelling the housing is sensed by the doctor, in order to solve the problems mentioned above.

### SOLUTION TO PROBLEM

An injection tool for an endoscope according to a first aspect of the present invention includes an outer tube, an operation unit main body which supports a base end of the outer tube, a needle body, an inner tube which supports a base end of the needle body, and a slider which supports a base end of the inner tube,
in which the inner tube is accommodated so as to be movable from the base end side of the operation unit main body into the outer tube, and the distal end side of the slider is accommodated in the operation unit main body so as to be movable at a predetermined stroke, thereby projecting and setting the needle body from the distal end side of the outer tube by operating the slider to move forward and backward with respect to the operation unit main body, and injecting a treatment liquid supplied to an inner portion from the base end of the slider into an affected area from the needle body when puncturing to the affected area in a protruding state of the needle body,
wherein the slider has an arm in which a locked protrusion is formed and which extends to a distal end side with an elastic restoring force,
wherein the operation unit main body has a guide groove for guiding the slider in a forward and backward moving direction, and a locking protrusion which is disposed in the base end side of the guide groove and prevents the slider from moving to the distal end side by positioning a locked protrusion between itself and the base end of the guide groove,
wherein the locking protrusion and the locked protrusion are configured to come into contact with each other so that the slider is transferable from a first state in which the movement to the distal end side of the slider is prevented, to a second state in which the locked protrusion of the slider gets over the locking protrusion according to the deformation due to the elastic restoring force of the arm to cancel the prevention of the movement when the slider is moved to the distal end side, and
wherein an operation feeling is generated by increase and decrease of an operation force when the locked protrusion gets over the locking protrusion.

Further, according to a second aspect of the present invention, there is provided the injection tool for the endoscope according to the first aspect,
wherein the arm is a pair of arms which extend to the distal end side with the elastic restoring force,
wherein a first protrusion protruding in an elastic direction is formed in a distal end of one arm of the pair of arms, and a second protrusion protruding in an elastic direction and including the locked protrusion is formed in a distal end of the other arm,
wherein the second protrusion of the other arm is fitted into the guide groove and is guided in the forward and backward moving direction,
wherein the operation unit main body is open a locking hole for first protrusion which is open closer to the distal end side than the guide groove and is fitted to the first protrusion, and
wherein the first protrusion is fitted to the locking hole for first protrusion by moving the slider further to the distal end from the second state, and an operation feeling is generated by increase and decrease of an operation force when transferring to a third state of locking a state in which the needle body protrudes out of the distal end of the outer tube.

Further, according to a third aspect of the present invention, there is provided the injection tool for the endoscope according to the second aspect,
wherein the locking protrusion is disposed in both inner surfaces of the guide groove in a width direction, and
wherein the locked protrusion is disposed to protrude toward the width direction of the guide groove in the second protrusion.

Further, according to a fourth aspect of the present invention, there is provided the injection tool for the endoscope according to the third aspect,
wherein the locked protrusion and the locking protrusion are formed into a semispherical shape.

### EFFECT OF INVENTION

The present invention can provide the injection tool for the endoscope which is locked in such a manner as to cancel the state in which the needle body protrudes out of the outer tube when injecting the treatment liquid from the puncture, the operation feeding at this locking time is sensed by the doctor, it is possible to maintain and cancel the state in which the needle body is housed within the outer tube before the puncture and after injecting the treatment liquid, and the operation feeling when operating for locking can be sensed by the doctor, thereby achieving the object of the present invention.

### BRIEF DESCRIPTION OF DRAWINGS

Fig. 1 is a plan view showing an outer appearance structure of disassembly and assembly in an injection tool for an endoscope according to an embodiment of the present invention.

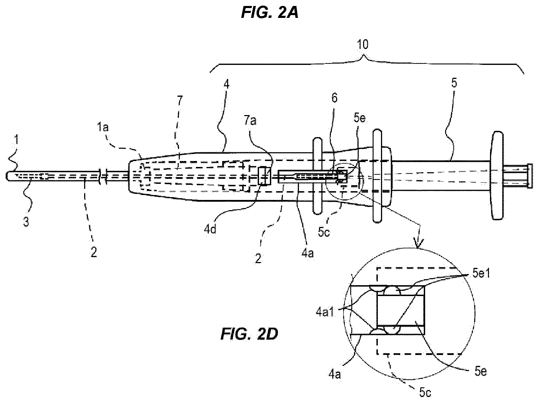

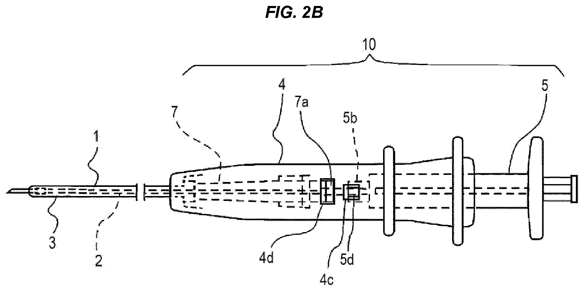

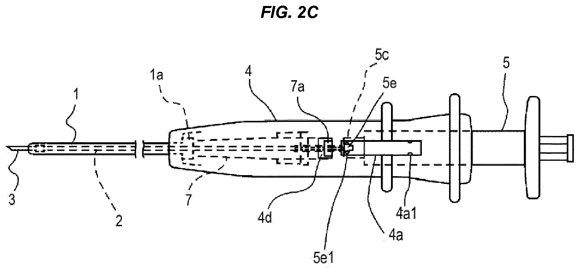

Figs. 2A to 2D show an overall outer appearance structure of the injection tool for the endoscope according to the embodiment of the present invention, in which Fig. 2A is a front elevational view showing a state in which a slider is at a position where the slider is retracted to a base end side and a needle body is housed within an outer tube, Fig. 2B is a view showing a state in which the injection tool for the endoscope is at a position where the slider is pushed from the state shown in Fig. 2A and the needle body is rotated at 180 degrees around a protruding center line from the outer tube, Fig. 2C is a front elevational view showing a state in which the slider is pushed from the state shown in Fig. 2A and the needle body protrudes out of the outer tube, and Fig. 2D is an enlarged view obtained by picking up a portion rounded by a circle in Fig. 2A.

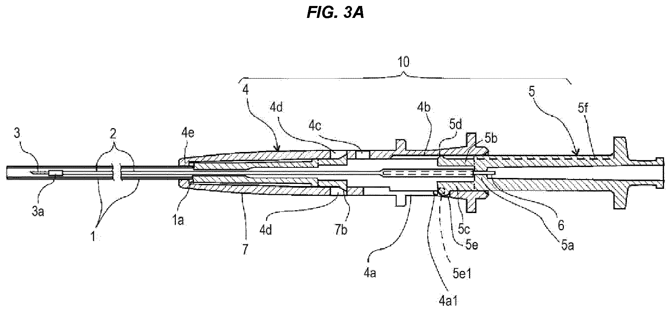

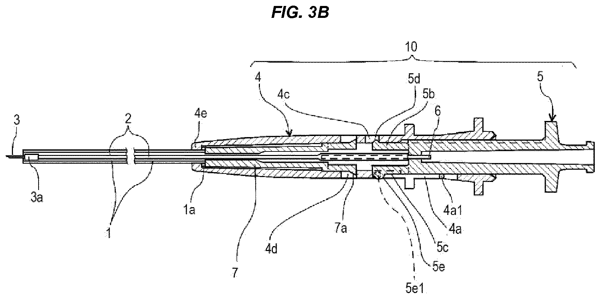

Figs. 3A and 3B are an overall vertical cross sectional view of the injection tool for the endoscope according to the embodiment of the present invention, in which Fig. 3A is a cross sectional view in which the injection tool for the endoscope shown in Fig. 2A is rotated at 90 degrees, and an engagement state between a first protrusion and a locking hole for first protrusion and an engagement state between a second protrusion and a guide groove for second protrusion are visible, and Fig. 3B is a cross sectional view showing a state in which the slider is at a position where the slider is pushed from the state shown in Fig. 3A and the needle body protrudes out of the outer tube.
Figs. 4A and 4B are views showing an operation unit main body of the injection tool for the endoscope according to the embodiment of the present invention, in which Fig. 4A is a cross sectional view through a center line, and Fig. 4B is a view as seen from an arrow B-B shown in Fig. 4A.
Figs. 5A and 5B are views for describing the slider of the injection tool for the endoscope according to the embodiment of the present invention, in which Fig. 5A is a front elevational view, and Fig. 5B is a view as seen from an arrow B-B shown in Fig. 5A.
Figs. 6A and 6B are views for describing an inner guide of the injection tool for the endoscope according to the embodiment of the present invention, in which Fig. 6A is a top elevational view, and Fig. 6B is a front elevational view.

### DESCRIPTION OF EMBODIMENTS

A description will be given below of a treatment tool for an endoscope according to an embodiment of the present invention with reference to the accompanying drawings. In the following description, a side where a distal end treatment unit (a needle bod) is positioned in an assembled state is called as a distal end side, a side where an operation unit is positioned is called as a base end side, and a direction of a segment connecting the distal end side and the base end side is called as an axial direction.

### [Basic Structure]

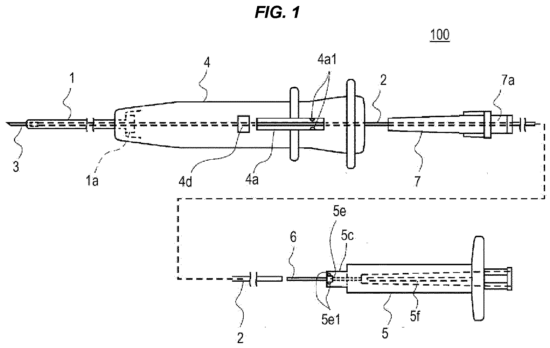

An injection tool for an endoscope 100 according to an embodiment of the present invention is provided with a first assembly including an elongated cylindrical outer tube 1 which is inserted into a channel of the endoscope, and an operation unit main body 4 in which the outer tube 1 is attached to a distal end thereof and which is provided for an operator to grip, and a second assembly including an inner tube 2 in which a needle body 3 is attached in a distal end thereof and which passes through the outer tube 1, an inner guide 2 (refer to Fig. 2A) through which the inner tube 2 passes and which is pushed into an inner portion of the operation unit main body 4, and a slider 5 which is connected to a base end side of the inner tube 2, is disposed in a proximal end side of the inner guide 7, and moves in an axial direction with respect to the operation unit main body 4, as shown in Fig. 1 mainly showing an outer appearance of an outline state for assembly and disassembly.

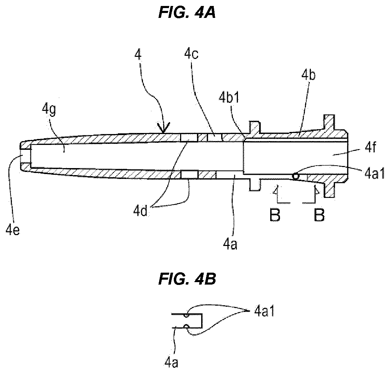

The operation unit main body 4 is formed into such a tubular shape that an inner guide 7 and a slider 5 can be inserted into an inner portion thereof, a cross section thereof being shown in Fig. 4A, and is provided with a distal end reduced diameter tube part 4e which is diameter reduced to a distal end side, a locking opening 4d which is used for locking the inner guide 7 mentioned later approximately at the center of a tubular side surface, and a guide groove for second protrusion 4a which extends to a base end side from a position of the locking opening 4d and guides movement of a second protrusion 5e of the slider. Details of the opening unit main body 4 will be described later.

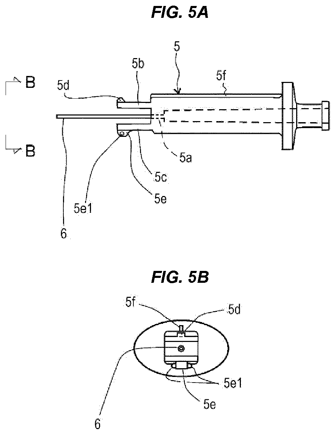

The slider is formed into an approximately elongated cubic shape in which a tube part is open in an inner portion thereof, as shown in Figs. 5A and 5B, is open to a base end side an injection port which can inject a treatment liquid such as a physiological saline solution and a drug solution, and is provided with a first arm 5b (the first arm 5b is disposed in a back side in Fig. 1 and is not illustrated) and a second arm 5c which extend like a bifurcated shape at symmetrical position in a diametrical direction of the distal end and respectively protrude a first protrusion 5d and a second protrusion 5e in a radial direction with an elastic restoring force. A detailed structure thereof will be mentioned later.

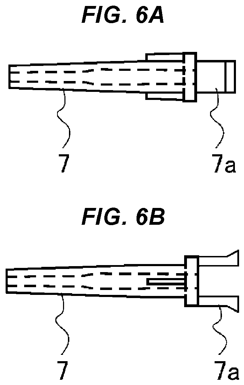

The inner guide 7 is formed into such a tubular shape that the inner tube 2 can be inserted into an inner portion thereof, a cross section thereof being shown in Fig. 6, and is provided in a base end side thereof with a pair of locking pawls 7a which extend in a bifurcated manner like an arm with a flexibility for fitting to a pair of locking openings 4d open to a side surface of the operation unit main body 4. The inner guide 7 is inserted into an innermost end of the operation unit main body 4 as shown in Fig. 1, and is adapted to bring the outer tube 1 into contact with a stopper 1a to hold in a distal end surface thereof and fit the locking pawls 7a to the locking openings 4d of the operation unit main body 4 to be fixed to the operation unit main body 4. A detailed structure thereof will be mentioned later.

As mentioned above, the injection tool for the endoscope 100 according to the present embodiment is structured such that the inner tube 2 is accommodated within the outer tube 1, the distal end side of the slider 5 is accommodated in the operation unit main body 4 in such a manner as to be movable at a predetermined stroke (allow the slider 5 to freely slide in an axial direction with respect to the operation unit main body 4 into which the inner guide 7 is inserted), and the operation unit main body 4 and the slider 5 are configured to be relatively locked and unlocked in a state in which the needle body 3 is housed within the outer tube 1.

A detailed structure of the injection tool for the endoscope 100 is constructed by the following section, as shown in Figs. 2A and 3A which show a state in which the slider 5 is inserted into the operation unit main body 4.
(1) The operation unit main body 4 which is formed into a tubular shape for being gripped by an operator includes the distal end reduced diameter tube part 4e obtained by diameter reducing a cylinder in the distal end side, the locking opening 4d which is opened for locking the inner guide 7, and the guide groove for second protrusion 4a which is opened for allowing the second protrusion 5e of the slider 5 to slide (details will be shown in Fig. 4).
(2) The elongated cylindrical outer tube 1 which is inserted from the base end side opening of the operation unit main body 4, has the flange-shaped stopper 1a in the base end side, brings the stopper 1a into contact with the distal end reduced diameter tube part 4e of the operation unit main body 4, and is fixed to the operation unit main body 4.
(3) The inner guide 7 which is inserted from the base end side opening of the operation unit main body 4, and in which the distal end side coming into contact with the stopper 1a of the outer tube 1 is clamped by the distal end reduced diameter tube part 4e of the operation unit main body 4 and a pair of locking pawls 7a are locked by a pair of locking openings 4d of the operation unit main body 4 (details will be shown in Fig. 6).
(4) The slider 5 which is provided with the first arm 5b and the second arm 5c protruding the first protrusion 5d and the second protrusion 5e in the radial direction to the distal end side and having the elastic restoring force of the arm, and in which approximately a half portion in the distal end side is inserted from the base end opening of the operation unit main body 4, and the second protrusion 5e disposed in the distal end is guided by the guide groove for second protrusion 4a of the operation unit main body 4 to be fitted to be slidable at a predetermined stroke (details will be shown in Fig. 5).
(5) An insert pipe 6 which is fitted and fixed to the distal end reduced diameter tube part 5a of the slider 5 in the base end and extends to the distal end side of the slider 5 before the inner guide 7 (refer to Fig. 3A).
(6) The inner tube 2 which is fixed and connected in a state in which the inner pipe 6 is forcibly inserted into the base end so that an inner diameter is enlarged and has a desired length.
(7) The tubular needle body 3 which is fixed and connected in the distal end thereof to the distal end of the inner tube 2.

In the present invention, the protrusion elastically supported to the distal end side of the slider 5 is called as a locked protrusion, and the protrusion fixed to the operation unit main body 4 and coming into contact with the locked protrusion and stopping the locked protrusion is called as a locking protrusion.

In the injection tool for the endoscope 100, the base end side of the second protrusion 5e of the slider 5 comes into contact with the inner surface in the base end side of the guide groove for second protrusion 4a of the operation unit main body 4 as shown in Figs. 2A and 3A when the slider 5 is in the shallowest fitting state to the operation unit main body 4 (when the slider 5 is at a position where it is retracted closest to the base end side of the operation unit main body 4), thereby preventing the slider 5 from falling to the base end side, and a locked protrusion 5e1 and the locking protrusion 4a1 come into contact with each other at a position where the locked protrusion 5e1 is between the base end side of the guide groove for second protrusion 4a and the locking protrusion 4a1, thereby preventing the slider 5 from moving to the distal end side, so that a state in which the needle body 3 is retracted into the distal end of the outer tube 1 is maintained.

Further, in the injection tool for the endoscope 100 according to the present embodiment, when the slider 5 is in the deepest fitting state to the operation unit main body 4 (when the slider 5 is at a position where it is pushed deepest to the operation unit main body 4), the first protrusion 5d of the slider 5 fits to a locking hole for first protrusion 4c of the operation unit main body 4 as shown in Figs. 2B, 2C and 3B, so that the slider 5 and the operation unit main body 4 are relatively fixed, and a state in which a desired length portion of the needle body 3 protrudes out of the distal end is held. Therefore, the injection tool for the endoscope 100 can inject out the treatment liquid injected into the inner portion from the base end of the slider 5 from the inner tube 2.

As mentioned above, the injection tool for the endoscope 100 according to the present embodiment can allow the operator (the doctor or the technologist) to sense the operation feeling and the fitting sound caused by increase and decrease of an operation force (force for moving forward and backward the slider 5 with respect to the operation unit main body 4), at the locking time when the slider 5 moves to the deepest fitting position to the operation unit main body 4, and at the unlocking time when the pulling movement starts.

A description will be in detail given below of a structure of each of the parts.

### [Outer Tube 1]

The outer tube 1 is formed into an elongated shape having a circular cross sectional tube passage by a plastic material having a flexibility, and is formed such that a passage inner diameter is thicker at a desired dimension than an outer diameter of the inner tube 2, for example, as shown in each of Figs. 3A and 3B. Further, the outer tube 1 has the flange-like stopper 1a expanding to an outer peripheral direction in the base end side for attaching to the operation unit main body 4 mentioned later. The outer tube 1 is constructed, for example, by polyether ether ketone (PEEK) resin or polytetrafluoroethylene (PTFE).

### [Inner Tube 2]

The inner tube is inserted into the outer tube 1 so as to be movable forward and backward, and is formed into a hollow elongated shape with a flexibility for feeding the treatment liquid. The inner tube 2 is constructed by the same material as the outer tube 1.

### [Needle Body 3]

The needle body 3 is formed into a tubular shape and is cut diagonally in the distal end portion. An outer diameter of the needle body 3 is smaller than an inner diameter of the inner tube 2. The base end side of the needle body 3 and the distal end of the inner tube 2 are fitted therebetween by a joint pipe 3a, so that a connection strength between the needle body 3 and the inner tube 2 is held great. The needle body 3 is constructed by a hard metal material or a hard plastic material, for example, a stainless steel. In the needle body 3, the inner diameter of the distal end portion of the outer tube 1 is diameter reduced with a step surface. Thus, the joint pipe 3a cannot pass through the distal end reduced diameter portion of the outer tube 1 and comes into contact with the step surface of the reduced diameter portion in the distal end even when the needle body 3 protrudes out of the outer tube 1. Then, the needle body 3 stops. The joint pipe 3a is not necessarily required. In this case, the distal end surface of the inner tube 2 should be structured such as not to pass through the distal end reduced diameter part of the outer tube 1 and to come into contact with the step surface of the distal end reduced diameter part and stop.

### [Operation Unit 10]

The operation unit 10 is constructed by the hollow operation unit main body 4, and the slider 5 which is fitted its distal end side from the opening portion in the base end side of the operation unit main body 4 so as to be slidable and undetachable.

### [Operation Unit Main Body 4]

The operation unit main body 4 is formed into a tubular shape having a slider accommodating hole 4f for inserting the slider 4 from the base end side, an inner guide accommodating hole 4g for housing the inner guide 7 in an inner portion, and a distal end reduced diameter tube part 4e obtained by diameter reducing a distal end side of the inner guide accommodating hole 4g, as shown in Figs. 4A and 4B. The operation unit main body 4 has the guide groove for second protrusion 4a which is opened for guiding the second protrusion 5e of the slider 5 in an axal direction, a rib guide groove 4b which is formed for guiding a rib 5f provided in the slider 5 in a longitudinal direction (an axial direction), the locking hole for first protrusion 4c which is opened for fitting the first protrusion 5d provided in the slider 5 thereto, and a pair of locking openings 4d which are opened for attaching the inner guide 7 to the innermost within the operation unit main body 4.

The guide groove for second protrusion 4a is opened to the side surface of the operation unit main body 4 as a rectangular shape with a desired length, and is provided in the vicinity of a base end (a wall in a base end side of the groove) with a pair of locking protrusions 4a1 which protrudes to both inner surfaces in a width direction, and a position separating from the base end opening of the operation unit main body 4 at a desired dimension is called as an opening base end. A length from the base end opening of the operation unit main body 4 to the opening base end in the guide groove for second protrusion 4a is set in correspondence to a dimension that the slider 5 can stably slide to the operation unit main body 4. The length of the guide groove for second protrusion 4a is set in correspondence to a stroke that the joint pipe 3a comes close to the distal end reduced diameter part of the outer tube 1 from a position where the needle body 3 is retracted to the outer tube 1.

The rib guide groove 4b is formed inward from the base end opening of the operation unit main body 4 for guiding the rib 5f provided in the slider 5 in the longitudinal direction to the opposite side (inner side) in the peripheral direction to the guide groove for second protrusion 4a, and a step A in the innermost end of the groove is set in such a manner as to come into contact with a step B of the distal end of the rib 5f when pressing the slider 5 to the innermost with respect to the operation unit main body 4.

The locking hole for first protrusion 4c is opened closer to the distal end side than the rib guide groove 4b in such a manner that the first protrusion 5d provided in the slider 5 fits thereto when the slider 5 is pressed to the innermost with respect to the operation unit main body 4 and the needle body 3 protrudes out of the outer tube 1.

The pair of locking openings 4d are opened at two positions in the peripheral direction in the distal end side of the locking hole for first protrusion 4c for attaching the inner guide 7 to the innermost within the operation unit main body 4. In the pair of locking openings 4d, phases in the peripheral direction (position in the peripheral direction) coincide with each other with respect to the guide groove for second protrusion 4a and the locking hole for first protrusion 4c.

The distal end reduced diameter tube part 4e is formed in the distal end of the operation unit main body 4, has such an inner diameter that can pass the outer tube 1 barely, but cannot pass the flange-loke stopper 1a provided in the base end of the outer tube 1, and has such an inner diameter that can stably hold a desired length of the base end of the outer tube 1.

### [Slider 5]

The slider 5 is formed into a hollow shape as shown in Figs. 5A and 5B, forms in a base end opening portion an injection port which can inject the treatment liquid such as the physiological saline solution or the drug solution by the connection of a syringe barrel (not shown), and has the distal end reduced diameter tube part 5a having the reduced diameter in an outlet side of the injection port. The distal end reduced diameter tube part 5a is fitted thereto the insert pipe 5 for connecting the inner tube 2. The insert pipe 6 is made of the stainless steel or the hard plastic, and is fitted from the base end opening of the inner tube 2 in such a manner as to expand the inner diameter of the inner tube 2 to be fixed and connected to the inner tube 2.

Further, the slider 5 forms the first arm 5b and the second arm 5c which are separated like a bifurcated manner at the symmetrical position in the diametrical direction (the peripheral direction) in the distal end side, can be elastically deformed in the diametrical direction respectively and have the elastic restoring force, as shown in Fig. 5A. The first arm 5b has the first protrusion 5d which protrudes outward in a radial direction (a deformable direction by the elastic restoring force: elastic direction) from the distal end, and the second arm 5c has the second protrusion 5e which protrudes outward in the radial direction (the deformable direction by the elastic restoring force: elastic direction) from the distal end, as shown in Fig. 5B.

In the first protrusion 5d, the distal end of the first arm 5b elastically deforms inward by the elastic restoring force when the first protrusion 5d comes into contact with the inner wall of the operation unit main body 4. Further, the second protrusion 5e is provided with a pair of locked protrusions 5e protruding out of the side surface as shown in Fig. 5B, and comes into contact with the semispherical locking protrusion 4a1 of the operation unit main body 4 as shown in Fig. 2D. Therefore, in the slider 5, the needle body 3 is prevented from moving to the distal end side of the outer tube 1. The slider 5 is pressed further to the operation unit main body 4, and the distal end of the second arm 5c accordingly deforms inward, so that the locked protrusion 5e1 gets over the locking protrusion 4a1 so as to cancel the movement prevention.

Further, the slider 5 is provided with the rib 5f which is formed in a longitudinal direction at a position in the same peripheral direction as the first protrusion 5d and closer to the base end side than the first protrusion 5d, as shown in Fig. 5B.

In the slider 5, the second protrusion 5e comes into contact with the base end of the guide groove for second protrusion 4a (the base end side wall of the groove) in the operation unit main body 4, thereby preventing the slider 5 from falling from the operation unit main body 4, and the locked protrusion 5e1 of the slider 5 comes into contact with the locking protrusion 4a1 of the operation unit main body 4, thereby preventing the slider 5 from moving to the distal end side more, and preventing the needle body 3 from protruding out of the outer tube 1.

In the slider 5, when the rib 5f is fitted inside from the base end opening of the operation unit main body 4 so as to fit into the rib guide groove 4b of the operation unit main body 4, the step surface in the distal end of the rib 5f comes into contact with the step surface 4b1 (refer to Fig. 4) in the distal end of the rib guide groove 4b of the operation unit main body 4, and the second protrusion 5e engages with the guide groove for second protrusion 4a provided in the operation unit main body 4 and comes into contact with the distal end side of the guide groove for second protrusion 4a, thereby restricting a relative movement in a pressing direction with respect to the operation unit main body 4.

When the relative movement in the pressing direction of the slider 5 with respect to the operation unit main body 4 is restricted, the first protrusion 5d fits into the locking hole for first protrusion 4c provided in the operation unit main body 4 with the elastic restoring force of the first arm 5b with producing a clicking sound, as shown in Fig. 3B. Thus, in the injection tool for the endoscope 100 according to the present embodiment, the operator can confirm the locking state between the slider 5 and the operation unit main body 4, and the fact that the needle body 3 protrudes out of the outer tube 1.

The first protrusion 5d is protruded like a chevron shape so that the distal end protrudes in the peripheral direction. Owing to the chevron shape, the injection tool for the endoscope 100 according to the present embodiment prevents the fitting state (the locking state) from being cancelled since the chevron slope surface comes into contact with the base end side of the locking hole for first protrusion 4c even if the operator loses hold of the operation unit 10.

Further, in the injection tool for the endoscope 100 according to the present embodiment, the first protrusion 5d is disconnected from the locking hole for first protrusion 4c when the operator houses the needle body 3 within the inner tube 2. Therefore, the force of pressing the first protrusion 5d inward is generated between the chevron slope surface of the first protrusion 5d and the locking hole for first protrusion 4c by strongly pulling the slider 5 to the base end side direction with respect to the operation unit main body 4, and the first arm 5b bends inward. Thus, the first protrusion 5d is depressed into the operation unit main body 4 and can cancel the fitting state (the locking state) to the locking hole for first protrusion 4c.

Further, the slider 5 is restricted in the relative movement in the pulling direction with respect to the operation unit main body 4. More specifically, the slider 5 is restricted in the relative movement in the pulling direction with respect to the operation unit main body 4 and is prevented from being easily disconnected from the operation unit main body 4 since the second protrusion 5e engages with the guide groove for second protrusion 4a and comes into contact with the base end of the guide groove for second protrusion 4a.

### [Inner Guide 7]

As shown in Figs. 6A and 6B, the inner guide 7 is formed in such a manner that the inner tube 2 can be inserted into an inner portion thereof, and has a pair of locking pawls 7a in a base end side, the locking pawls 7a having a flexibility for fitting to a pair of locking openings 4d disposed in the operation unit main body 4 and expanding outward in a bifurcated manner to extend like an arm shape.

As shown in Figs. 6A and 6B, the inner guide 7 is provided with a pair of locking pawls 7a each of which is inserted in a base end side thereof into an innermost end from a base end opening of the operation unit main body 4 and comes into contact with the stopper 1a of the outer tube 1 in a distal end surface for fitting to the locking opening 4d of the operation unit main body 4 and locking. Widths of a pair of locking pawls 7a and a pair of locking openings 4d are set to be larger than widths of the locking hole for first protrusion 4c and the guide groove for second protrusion 4a for preventing a pair of locking pawls 7a from erroneously fitting to the locking hole for first protrusion 4c or the guide groove for second protrusion 4a when a pair of locking pawls 7a are installed (fitted to the locking openings 4d).

In the injection tool for the endoscope 100 having the basic structure mentioned above, the operator inserts it into a channel of an endoscope (not shown) previously inserted into a body cavity of a patient in a state in which the needle body 3 is retracted into the outer tube 1, thereby allowing the distal end side to protrude from the distal end of the endoscope near the affected area. Further, in the injection tool for the endoscope 100, when the operator pushes forward the slider 5 for the operation unit main body 4, the first protrusion 5d of the slider 5 fits to the locking hole for first protrusion 4c of the operation unit main body 4, the clicking sound is produced, the protrusion of the needle body 3 out of the distal end of the outer tube 1 can be confirmed, and the protruding state of the needle body 3 can be held (refer to Figs. 2B, 2C, and 3B).

Further, the slider 5 is prevented from moving further to the distal end side since the step surface in the distal end of the rib 5f in the slider 5 comes into contact with the step surface 4b1 in the distal end of the rib guide groove 4b in the operation unit main body 4, and the second protrusion 5c of the slider 5 comes into contact with the distal end of the guide groove for second protrusion 4a in the operation unit main body 4.

In the injection tool for the endoscope 100, the state in which the needle body 3 attached to the distal end of the inner tube 2 is protruded out of the distal end of the outer tube 1 is locked, and the operator punctures the needle body 3 to the submucosal layer in the affected area. In this state, the operator injects the treatment liquid such as the physiological saline solution or the drug solution into the submucosal layer in the affected area via the inner tube 2 and the needle body 3 from the syringe barrel connected to the slider 5, and forms the submucosal layer in a floated state, and resects it with a high frequency knife or a high frequency snare.

### [Characteristic Structure]

Subsequently, a description will be given of a characteristic structure of the injection tool for the endoscope 100 according to the embodiment of the present invention.

The injection tool for the endoscope 100 according to the present embodiment has the following functions as shown in Figs. 2A, 2D and 3A.
(1) In a state in which the slider 5 is inserted into the operation unit main body 4, and the slider 5 can slide along the guide groove (the guide groove for second protrusion 4a) of the operation unit main body 4 and is prevented from falling, the semispherical locked protrusion 5e1 disposed in the distal end side of the slider 5 comes into contact with a pair of semispherical locking protrusions 4a1 of the operation unit main body 4 and is held with respect to an edge in the base end side of the guide groove for second protrusion 4a. Thus, a state (a first state) in which the movement of the slider 5 is prevented is achieved, and the protrusion of the needle body 3 out of the outer tube 1 is prevented.
(2) When the operator further pushes the slider 5 into the operation unit main body 4 from the first state in which the protrusion of the needle body 3 is prevented, the locked protrusion 5e1 of the slider 5 gets over the locking protrusion 4a1 of the guide groove for second protrusion 4a by the elastic restoring force of the arm and cancels the prevention of the slider movement (a second state). When the slider 5 is slid to the inner position of the guide groove for second protrusion 4a, and the first protrusion 5d of the slider 5 is fitted to the locking hole for first protrusion 4c of the operation unit main body 4, the state in which the needle body 3 is protruded out of the inner tube 2 is locked (a third state). The action that the locked protrusion 5e1 gets over the locking protrusion 4a1 of the guide groove for second protrusion 4a is achieved by the inward movement of the locked protrusion 5e1 elastically supported by the arm due to the elastic deformation of the second arm 5c.

The sliding mentioned above can be set to an opposite direction, and the locking protrusion 4a1 and the locked protrusion 5e1 are preferably formed into the semispherical shape, however, are not limited to this.

Further, in the present embodiment, the description is given of the example in which two arms are provided by dividing the distal end side of the slider 5 in the bifurcated shape, one arm is applied to the function of preventing the needle body from protruding, and the other arm is applied to the function of locking the protrusion of the needle body. However, the present invention is not limited to this structure. A mechanism according to the other example may be provided with a locked protrusion which is elastically supported by a distal end of one arm extending from the distal end of the slider, and a guide groove for a protrusion in which locking protrusions are provided at two positions, that is, near the base end side and near the distal end side (terminal end), and may be adapted to prevent the protrusion of the needle body by the locking protrusion near the base end side and lock the protruding state of the needle body by the locking protrusion near the distal end side (terminal end).

Further, in the present embodiment, the description is given of the mechanism example including the locked protrusion 5e1 which protrudes out sideward (in the groove width direction) from the second protrusion 5e disposed in the second arm 5c of the slider 5 as shown in Fig. 5B. However, the mechanism according to the present invention is not limited to this structure. A mechanism according to the other example may employ, for example, a structure in which a locked protrusion (an elastically supported protrusion) elastically supported from a slider and an operation unit main body and a fixed locking protrusion (a fixed position protrusion) are provided without employing the locked protrusion protruding sideward, both the protrusions come into contact and prevent the needle body from protruding, and the elastically supported protrusion gets over the fixed position protrusion and cancels the movement prevention by further pressing the slider to the operation unit main body. The structure of the protrusion may employ the other structure, for example, may employ a structure constructed by combination of a slope surface and a protrusion with a circular arc curved surface which are inclined in the same direction and come into contact with each other.

As described above, the injection tool for the endoscope 100 according to the present embodiment is provided with the locked protrusion 5e1 in the elastically bending second arm 5c, and with the locking protrusion 4a1 near the base end side of the guide groove for second protrusion 4a. Therefore, the locked protrusion 5e1 gets over the locking protrusion 4a1 disposed within the guide groove for second protrusion 4a to move. At this time, whatever direction the locked protrusion 5e1 moves, the distal end side of the second arm 5c bends inward by the elastic restoring force of the arm and the locked protrusion 5e1 comes into slidable contact with the locking protrusion 4a1 while moving inward and gets over the locking protrusion 4a1.

The above-described getting-over is carried out in an extremely small distance and is sensed by the operator as an operation feeling (clicking feeling) associated with the force increase and decrease.

More specifically, in the injection tool for the endoscope 100 according to the present embodiment, the locked protrusion 5e1 gets over the locking protrusion 4a1 toward the base end side of the guide groove for second protrusion 4a, the locked protrusion 5e1 moves to the distal end side of the guide for second protrusion 4a1 by the elastic restoring force of the second arm 5c, and the first protrusion 5d of the slider 5 fits to the locking hole for first protrusion 4c of the operation unit main body 4. Thus, the locking state in which the needle body is protruded is formed, and it is possible to allow the operator to sense the fitting sound generated when fitting and the operation feeling caused by the increase and decrease of the operation force.

As mentioned above, in the injection tool for the endoscope 100 according to the characteristic structure, in the state in which the needle body 3 is housed within the outer tube 1, the second protrusion 5e comes to the base end position of the guide groove for second protrusion 4a and prevent the slider from moving. Therefore, it is possible to solve the problem in the prior art. More specifically, even in a case where the slider 5 is not held and the operation unit main body 4 is held by setting the operation unit main body 4 in the lower side and the slider 5 in the upper side, a case where the flange part of the operation unit main body 4 is hooked and the injection tool for the endoscope is suspended by setting the operation unit main body 4 in the lower side and slider 5 in the upper side for an easy treatment by the technologist during an operation, and the other case where something comes into conflict with the injection tool, the injection tool for the endoscope 100 according to the present embodiment can solve a problem generated by the fact that the needle body 3 comes out of the distal end of the outer tube 1 on its own. Further, it is possible to prevent the injection tool for the endoscope 100 from coming into contact with the other area than the affected area after the treatment and prevent the needle body 3 from coming into contact with the inner surface of the channel when pulling out the operation unit main body 4 from the channel of the endoscope.

Further, in the injection tool for the endoscope 100 according to the present embodiment, the locked protrusions 5e1 and 5e1 can move to the distal end side of the slider 5 so as to get over the locking protrusions locking protrusions 4a1 and 4a1 when a predetermined force is applied to the slider 5 in the pressing direction. Therefore, the needle body 3 can be protruded out of the outer tube 1 at a position where the outer tube 1 is corresponded to the affected area.

According to the injection tool for the endoscope 100 of the embodiment mentioned above, the needle body 3 is locked to be unlockable in the protruding state from the outer tube 1 when injecting the treatment liquid after the puncture, and the clicking sound and the operation feeling (clicking feeding) when locking are sensed by the doctor. In addition, the needle body 3 is prevented from moving even in a state in which the needle body 3 is housed within the outer tube 1 before the puncture and after the injection of the treatment liquid, and the clicking sound and the operation feeling (clicking feeling) can be sensed by the doctor when the movement is prevented and the movement prevention is cancelled.

### REFERENCE SIGNS LIST

1 outer tube
1a stopper
2 inner tube
3 needle body
3a joint pipe
4 operation unit main body
4a guide groove for second protrusion
4a1 locking protrusion
4b rib guide groove
4b1 step surface
4c locking hole for first protrusion
4d locking opening
4e distal end reduced diameter tube part
4f slider accommodating hole
4g inner guide accommodating hole
5 slider
5a distal end reduced diameter tube part
5b first arm
5c second arm
5d first protrusion
5e second protrusion
5e1 locked protrusion
5f rib
5f1 step surface
6 insert pipe
7 inner guide
7a locking pawl
10 operation unit
100 injection tool for endoscope

## Claims

1. An injection tool for an endoscope comprising:
an outer tube;
an operation unit main body which supports a base end of the outer tube;
a needle body;
an inner tube which supports a base end of the needle body; and
a slider which supports a base end of the inner tube;
wherein the inner tube is accommodated so as to be movable from the base end side of the operation unit main body into the outer tube, and the distal end side of the slider is accommodated in the operation unit main body so as to be movable at a predetermined stroke, thereby protruding and setting the needle body from the distal end side of the outer tube by operating the slider to move forward and backward with respect to the operation unit main body, and injecting a treatment liquid injected into an inner portion from the base end of the slider to an affected area from the needle body when puncturing to the affected arear in a protruding state of the needle body,
wherein the slider has an arm in which a locked protrusion is formed and which extends to a distal end side with an elastic restoring force,
wherein the operation unit main body has a guide groove for guiding the slider in a forward and backward moving direction, and a locking protrusion which is disposed in the base end side of the guide groove and prevents the slider from moving to the distal end side by positioning a locked protrusion between itself and the base end of the guide groove,
wherein the locking protrusion and the locked protrusion are configured to come into contact with each other so that the slider is transferable from a first state in which the movement to the distal end side of the slider is prevented, to a second state in which the locked protrusion of the slider gets over the locking protrusion according to the deformation due to the elastic restoring force of the arm to cancel the prevention of the movement when the slider is moved to the distal end side, and
wherein an operation feeling is generated by increase and decrease of an operation force when the locked protrusion gets over the locking protrusion.

2. The injection tool for the endoscope according to claim 1,
wherein the arm is a pair of arms which extend to the distal end side with the elastic restoring force,
wherein a first protrusion protruding in an elastic direction is formed in a distal end of one arm of the pair of arms, and a second protrusion protruding in an elastic direction and including the locked protrusion is formed in a distal end of the other arm,
wherein the second protrusion of the other arm is fitted into the guide groove and is guided in the forward and backward moving direction,
wherein the operation unit main body is open a locking hole for first protrusion which is open closer to the distal end side than the guide groove and is fitted to the first protrusion, and
wherein the first protrusion is fitted to the locking hole for first protrusion by moving the slider further to the distal end from the second state, and an operation feeling is generated by increase and decrease of an operation force when transferring to a third state of locking a state in which the needle body protrudes out of the distal end of the outer tube.

3. The injection tool for the endoscope according to claim 2,
wherein the locking protrusion is disposed in both inner surfaces of the guide groove in a width direction, and
wherein the locked protrusion is disposed to protrude toward the width direction of the guide groove in the second protrusion.

4. The injection tool for the endoscope according to claim 3,
wherein the locked protrusion and the locking protrusion are formed into a semispherical shape.
